# EUROPEAN PATENT APPLICATION

(11) **EP 0 870 492 A1**
(43) Date of publication of application: **14.10.1998**
(21) Application number: 97302475.5
(22) Date of filing: 11.04.1997
(51) Int. Cl.: A61K 7/16

(54) **Oral composition comprising aluminium hydroxide, silica and an anionic surfactant**

(71) Applicant: CARTER-WALLACE, INC., New York, N.Y. 10105 (US)
(72) Inventor: Fry, David R., Hythe, Kent CT21 5SY (US)
(74) Representative: W.P. Thompson & Co.

(57) **Abstract**

There is disclosed an oral composition comprising in an orally acceptable vehicle, an effective cleaning and polishing amount (about 20% to about 50% by weight) of an abrasive consisting of the combined abrasives aluminium hydroxide and hydrated silica, at least 10% by weight (based on the weight of the combined abrasives) of which is aluminium hydroxide and at least 10% by weight (based on the weight of the combined abrasives) of which is hydrated silica, in admixture with an anionic surfactant.

## Description

The present invention relates to dentifrice compositions. More particularly, the present invention relates to dentifrice compositions which may be liquids or pastes which exhibit superior cleaning and polishing properties. Specifically, the present invention is directed to a unique formulation containing a combination of agents for whitening and polishing teeth which maximize the whitening and polishing efficiency of the agents and which after use leave a mouth-feel similar to the feeling in the mouth usually experienced after cleaning by a dentist or dental hygienist.

More particularly, the present invention relates to a composition for cleaning and whitening the teeth based on the unique combination of aluminum hydroxide, hydrated silica and an anionic surfactant such as sodium dodecylbenzenesulfonate. Humectants, thickening agents, fluoridating agents, flavors, coloring agent, sweeteners and other conventional adjuvants, such as desensitizing agents and anticalculus agents, may also be included in the composition.

Abrasives are the solid materials added to dentifrice compositions whose functions are the removal of debris and stains from the teeth as well as the polishing of the tooth surface. An ideal abrasive material should maximize debris removal and effectively polish the teeth without causing undue abrasion to the hard tooth tissue.

Many different dentifrice compositions are known for cleaning and whitening the teeth. Of these known dentifrice compositions, many include high contents of water-insoluble abrasives which aid in removing plaque and retarding stain build-up on the teeth. However, since the ultimate goal of any oral hygiene regimen is preservation of the teeth, it is widely accepted that dentifrice compositions should include the least abrasive material necessary to remove plaque and stain.

Many materials have found utility as dental abrasives over the years, including:

Among the non-calcium cleaning and polishing agents, aluminum hydroxide, also known as aluminum hydrate and hydrated alumina, has found wide acceptance as a dental abrasive beginning from about 1926 when it was claimed in toothpaste compositions, i.e. Swedish Patent No. 69,077, followed by United States Patents Nos. 2,010,910; 2,222,969 and 2,550,207.

Aluminum hydroxide has found particular use in dentifrice formulations which contain ingredients, such as fluorides, that may be incompatible or difficulty compatible with the widely used calcium phosphates, calcium carbonate and sodium bicarbonate abrasives. The aluminum hydroxide abrasives generally have a particle size of from about 0.025 microns to about 50.0 microns.

Silica abrasives are another non-calcium abrasive which have found wide use in dentifrice compositions. The silica abrasives are polishing materials which have an average particle size ranging from about 0.1 to about 30 microns, generally between about 5 and 15 microns. The material can be precipitated silica or silica gels such as the silica xerogels described in U.S. Patent 3,862,307 and U.S. Patent 3,538,230. Widely used precipitated silica materials are those marketed by J.M. Huber Corporation under the tradename "Zeodent." Silica, like alumina, is more compatible with the components of dentifrice compositions such as fluorides than are the widely used calcium phosphate, calcium carbonate and sodium bicarbonate abrasives.

Among the most widely used abrasive and polishing components in dentifrice compositions are the calcium pyrophosphates such as those described in U.S. Patent 3,112,247 and the dibasic calcium phosphates. Calcium pyrophosphate prepared by the thermal decomposition of dibasic calcium phosphate has been used in toothpaste compositions for upwards of seventy five years.

Likewise, dicalcium phosphate has been widely used as a polishing agent in dentifrice compositions, however, it must be stabilized against spontaneous decomposition and/or hydrolysis particularly when used in conjunction with a source of fluoride ions such as monofluorophosphate.

While not wishing to be bound by any theory, minor amounts of calcium based materials may be added to the compositions of the present invention in order to provide a source of calcium mineral which upon absorption may improve the bone and tooth strength of the user.

According to the present invention, it has been surprisingly discovered that the combination of aluminum hydroxide, hydrated silica and an anionic surfactant such as sodium dodecylbenzenesulfonate as cleaning and polishing agents in dentifrice compositions maximizes the whitening and polishing efficiency and the grittiness image of the compositions providing the feeling in the mouth similar to that achieved after cleaning by a dentist or dental hygienist.

Preferably the anionic surfactant is also an antibacterial agent. Sodium dodecylbenzenesulfonate is also an antibacterial agent.

Moreover, the present invention is based upon the discovery that the unique combination of the whitening and polishing agents aluminum hydroxide and hydrated silica with an antibacterial detergent sodium dodecylbenzenesulfonate, which reduces surface tension and increases cleaning effect, yields a synergistic combination which maximizes the whitening and polishing efficiency of the abrasive material.

Preferably the antibacterial detergent is also an anionic surfactant.

The dentifrices of this invention generally include an orally acceptable vehicle (or suitable dentrifice carrier).

The dentifrices of the present invention may additionally contain a fluoridating agent to aid in preventing dental caries. Many fluoridating agents suitable for use in dentifrice compositions are known. Among these are sodium, potassium, lithium or ammonium fluorides, organic amine fluorides, monofluorophosphate salts such as sodium, potassium, lithium and ammonium monofluorophosphate, sodium fluorosilicate, sodium fluorozirconate, and other materials well known to those skilled in the art. The fluoridating agents are present in an effective but non-toxic amount, e.g. in amounts of up to about 2.0%, and preferably in an amount to provide a fluoride level equivalent to about 1000 ppm theoretical plus a 10% excess of fluoride ion.

Suitable surfactants which impart significant antibacterial action to the composition are the anionic surfactants, preferably sodium dodecylbenzenesulfonate as well as the sulfates of long chain (C₈-C₁₈) alcohols, e.g. sodium lauryl sulfate or sodium tridecyl sulfate, the sulfates or sulfonates of monoglycerides, e.g. sodium lauroyl glyceryl or sodium coconut monoglyceride sulfonate; the sulfonates of succinic esters, e.g. sodium dioctyl sulfosuccinate; the alkyl sulfoacetates such as sodium lauryl sulfoacetate or sodium coconut sulfoacetate; the salts of sulfoacetic acid amidified with amino ethyl long chain fatty acid esters such as sodium sulfocolaurate; the amides formed from higher fatty acids with short chain amino acids such as sodium lauroyl sarcosinate or sodium methyl lauroyl tauride and soaps such as the sodium, potassium or triethanolamine salts of fatty acids.

The dentifrice composition of the present invention in addition to the combined abrasives, fluoride and antibacterial detergent may include other ingredients customarily found in dentifrice compositions.

The formulation of the invention may include humectants and thickeners. Examples of humectants include glycerol, propylene glycol, sorbitol, polyethylene glycols (generally of formula H(OC₂H₄)ₙOH), and other materials known to those skilled in the art. The humectant may be present in the toothpaste in an amount of up to about 35%, preferably in an amount of about 8 to 25%, by weight of the dentifrice formulation. Suitable thickeners include cellulose gum, sodium carboxymethylcellulose, xanthan gum, methyl cellulose, hydroxethyl cellulose, carrageen, gum karaya, gum tragacanth, gum arabic, colloidal complex magnesium aluminum silicates (e.g. "Veegum", manufactured by R.T. Vanderbilt Co.) and sodium alginate. The thickener is incorporated in the formulation in an amount of up to about 3%, preferably within the range of about 0.3 to 1.5%, by weight of the dentifrice formulation.

In the dentifrice compositions of the present invention, the liquid vehicle may comprise water and a humectant typically in an amount ranging from about 10% to about 90% by weight of the preparation. Glycerine, propylene glycol, sorbitol, polypropylene glycol and/or polyethylene glycol (e.g. 400-600) exemplify suitable humectants/carriers. Also advantageous are liquid mixtures of water, glycerine and sorbitol.

Suitable flavoring and coloring agents include the flavoring oils, for example, peppermint, spearmint, menthol, wintergreen, clove, cinnamon, lemon, orange, methylsalicylate, licorice, or eucalyptus. The flavoring agent may be present in the dentifrice in an amount up to about 5.0% preferably in an amount within the range of about 0.3 to 2.0% by weight of the toothpaste. Useful coloring agents include FD&C Blue #1, Yellow #10 and titanium dioxide. Suitable sweeteners include sodium saccharin, sodium or calcium cyclamate, aspartame, and other sweeteners known to those skilled in the art. The sweetener may present in the toothpaste in an amount of up to about 5.0%, preferably about 0.3 to 2.0%, by weight of the toothpaste. It should be noted that humectants, e.g. sorbitol, may sweeten the formulation to some degree. However, the amount of humectant present in the composition not included in the range of sweetener set forth above.

The preferred dentrifice formulations of the present invention comprise from about 20% to about 50% (more preferably from about 27% to about 33%) by weight of combined abrasives at least 10% by weight of the combined abrasives being hydrated silica having a particle size of from 8 to 10 microns, and at least about 10% by weight of the combined abrasive being aluminium hydroxide having a particle size of from about 10 to 40 microns, and from about 1% to about 5%, preferably up to about 2% of an anionic surfactant, preferably sodium dodecylbenzenesulfonate.

The formulations of the present invention are prepared by well known methods which generally comprise first mixing a thickener if utilized with a humectant. A water solution containing sweetener, surfactant and fluoride is added to the thickener/humectant mixture, then the abrasives are stirred into the mixture. Finally, the flavor oil is added. The composition is then deaerated under vacuum to release trapped gases.

A number of desensitizing agents are available for the treatment of hypersensitive teeth. They include dibasic sodium citrate, potassium nitrate, strontium chloride and formaldehyde. The preferred material is potassum nitrate. The desensitizing agent may be present from 2.5% to 10.0%, with the preferable level of 5.0%.

Soluble pyrophosphates have been shown through several large scale studies to demonstrate their usefulness as inhibitors of calculus formulation. The ingredients employed as an anticalculus agent is specifically a combination of tetrapotassium pyrophosphate and tetrasodium pryophohphate at a range of 3.0% to 7.0% total. The preferable total level is 5.9% by weight.

The following examples illustrate particularly preferred embodiments of the dentifrice formulations of the present invention. In all examples, all parts and percentages are given by weight of the total composition.

### EXAMPLE 1

| Ingredient | % (w/w) |
|---|---|
| Sorbitol Solution 70% | 37.75 or q.s. |
| Hydrated Silica | 22.00 |
| Aqua | 10.00 |
| Glycerin | 13.00 |
| Aluminum Hydroxide | 10.00 |
| Sodium Dodecylbenzenesulfonate | 1.75 |
| PEG-12 | 1.5 |
| Aroma 2105 | 1.00 |
| Sodium Monofluorophosphate | 0.75 |
| Cellulose Gum | 0.40 |
| Trisodium Phosphate | 0.35 |
| Sodium Phosphate | 0.30 |
| Sodium Saccharin | 0.20 |
| Titanium Dioxide | 1.00 |

### EXAMPLE 2

| Ingredient | % (w/w) |
|---|---|
| Aqua | 22.526 or q.s. |
| Aluminum Hydroxide | 20.000 |
| Glycerin | 13.000 |
| Hydrated Silica | 13.000 |
| Sorbitol Solution 70% | 17.000 |
| Calcium Pyrophosphate | 7.000 |
| Sodium Dodecylbenzenesulfonate | 1.750 |
| PEG-12 | 1.300 |
| Aroma | 1.000 |
| Sodium Monofluorophosphate | 0.790 |
| Cellulose Gum | 0.350 |
| Sodium Saccharin | 0.170 |
| Sodium Fluoride | 0.110 |
| Titanium Dioxide | 2.000 |
| FD&C Blue #1 | 0.002 |
| FD&C Yellow #10 | 0.002 |

### EXAMPLE 3

| Ingredient | % (w/w) |
|---|---|
| Sorbitol Solution 70% | 37.75 or q.s. |
| Hydrated Silica | 22.00 |
| Potassium Nitrate | 5.00 |
| Aqua | 10.00 |
| Glycerin | 13.00 |
| Aluminum Hydroxide | 5.00 |
| Sodium Dodecylbenzenesulfonate | 1.75 |
| PEG-12 | 1.5 |
| Aroma 2105 | 1.00 |
| Sodium Monofluorophosphate | 0.75 |
| Cellulose Gum | 0.40 |
| Trisodium Phosphate | 0.35 |
| Sodium Phosphate | 0.30 |
| Sodium Saccharin | 0.20 |
| Titanium Dioxide | 1.00 |

Based on the results of a Pellicle Cleaning (whitening) Study conducted at a leading U.S. Dental University (Study No 96-113), a formulation very similar to that described in Example 1 had a higher Pellicle Cleaning (Whitening) Ratio than did comparable, competitive whitening products currently marketed in Germany. It should be noted that the abrasivity (RDA) is comparable to other typical daily use dentifrice products which is also below the industry safety standard.

It should be understood that the above Examples are illustrative of the best mode only of the invention herein disclosed. Given the present disclosure, it is anticipated that numerous variations will occur to those skilled in the art. A latitude of modification, substitution and change is intended and in some instances, some features of the invention will be employed without a corresponding use of other features. Accordingly, it is intended that the spirit and scope of the invention disclosed herein should be limited only by the following claims:

## Claims

1. An oral composition comprising in an orally acceptable vehicle, an effective cleaning and polishing amount (about 20% to about 50% by weight) of an abrasive consisting of the combined abrasives aluminium hydroxide and hydrated silica, at least 10% by weight (based on the weight of the combined abrasives) of which is aluminium hydroxide and at least 10% by weight (based on the weight of the combined abrasives) of which is hydrated silica, in admixture with an anionic surfactant.

2. An oral composition as claimed in claim 1 in which said anionic surfactant is sodium dodecylbenzenesulfonate.

3. An oral composition as claimed in claim 1 or 2, further containing a fluoridating agent in an amount to provide a fluoride level of 1000 to 1500 ppm.

4. An oral composition as claimed in any one of the preceding claims comprising from 13% to 22% by weight hydrated silica, from 5% to 20% by weight aluminium hydroxide, in admixture with an anionic surfactant and sodium fluoride.
